Europäisches Patentamt
European Patent Office
Office européen des brevets

(19)

(11) Veröffentlichungsnummer: **0 392 423 A2**

(12) # EUROPÄISCHE PATENTANMELDUNG

(21) Anmeldenummer: **90106764.5**

(22) Anmeldetag: **09.04.90**

(51) Int. Cl.$^5$: **C07D 257/02, C07F 9/38, A61K 49/02, A61K 43/00**

(30) Priorität: **11.04.89 DE 3911816**

(43) Veröffentlichungstag der Anmeldung: **17.10.90 Patentblatt 90/42**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Mäcke, Helmut, Dr.**
**Bergfriedweg 7**
**D-7850 Lörrach(DE)**

(54) **Substituierte 1,4-7-10-Tetraazacyclotridecane, Verfahren zu deren Herstellung sowie Verwendung derselben zur Markierung von Substanzen mit Radionukliden.**

(57) Die Erfindung betrifft 1,4,7,10-Tetraazacyclotridecane der Formel I

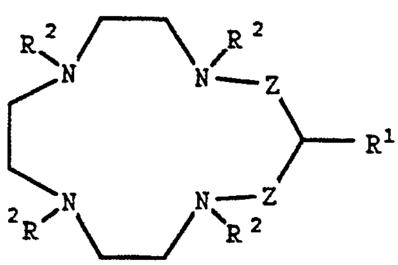

(I)

in der $R^1$, $R^2$ und Z die angegebenen Bedeutungen haben. Die erfindungsgemäßen Verbindungen eignen sich zur Markierung von Substanzen mit Radionukliden, insbesondere radioaktivem Technetium, Rhenium oder Indium.

Indium-Austausch gegen Transferrin in humanem Serum

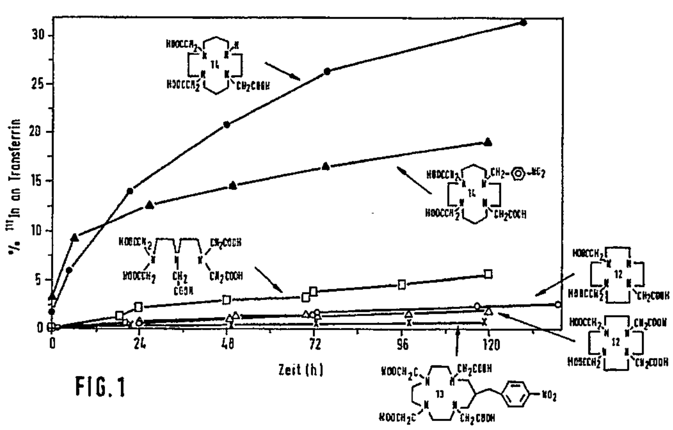


FIG.1

EP 0 392 423 A2

# Substituierte 1,4,7,10-Tetraazacyclotridecane, Verfahren zu deren Herstellung sowie Verwendung derselben zur Markierung von Substanzen mit Radionukliden

Gegenstand der Erfindung sind substituierte 1,4,7,10-Tetraazacyclotridecane, die sich zur Markierung von Substanzen mit Radionukliden, insbesondere radioaktivem Technetium, Rhenium- oder Indium eignen.

Die Anwendungsmöglichkeiten von Radionukliden in der Technik sind sehr vielfältig. Sie erstrecken sich beispielsweise auf die Prüfung einer Durchmischung, auf die verdünnungsanalytische Bestimmung von Mengen oder Volumina, auf die Messung der Strömungsgeschwindigkeit und Aufnahme der Verweilzeit an kontinuierlich arbeitenden Produktionsanlagen.

Meist genügt jedoch nicht die bloße Beimischung eines radioaktiven Nuklids, sondern beispielsweise bei der "Verfolgung" einer bestimmten Komponente eines Systems, muß das radioaktive Nuklid physikalisch oder besser noch chemisch an mindestens eine Verbindung der zu untersuchenden Komponente gekoppelt werden und dies nach Möglichkeit ohne die physikalischen und chemischen Eigenschaften der betreffenden Verbindung zu beeinflussen.

In den vergangenen Jahren wuchs das Verlangen, chemische Verbindungen mit radioaktiven Nukliden zu markieren. Vor allem im Bereich der medizinischen Diagnostik, wo pathologische Zustände bereits durch Substanzen angezeigt werden können, die im Organismus nur in ppm oder gar in noch geringeren Konzentrationen vorkommen, kann man heute nicht mehr auf radioaktiv markierte Substanzen verzichten. Insbesondere Technetium-99m ist wegen seiner günstigen physikalischen Eigenschaften (Fehlen einer Korpuskularstrahlung, $\gamma$-Energie von 140 keV und Halbwertszeit von 6 Stunden) und der damit verbundenen geringen Strahlenbelastung zum wichtigsten Radionuklid in der nuklearmedizinischen Diagnostik geworden.

Technetium-99m, das sich aus Nuklidgeneratoren gewinnen läßt, liegt zunächst als Pertechnetat vor, das in dieser Form, z.B. für die Schilddrüsen- und Hirnszintigraphie geeignet ist. Die Szintigraphie anderer Organe mittels Technetium-99m gelingt mit Hilfe bestimmter "Transportsubstanzen", die einerseits in der Lage sind Technetium zu binden, und andererseits das Radionuklid im Zielorgan mit hoher Selektivität anzureichern. Zur Markierung der organspezifischen "Transportsubstanz" mit Technetium-99m muß das aus dem Nuklidgenerator eluierte Pertechnetat zuerst in eine niedrigere Oxidationsstufe überführt werden. In dieser reduzierten Form bildet Technetium mit der organspezifischen Substanz mehr oder weniger stabile Verbindungen. Für die Knochenszintigraphie werden z.B. Tc-99m-Phosphorsäure-Derivate, vor allem organische Phosphonsäuren, eingesetzt. So liegt in der im Europäischen Patent 2485 beschriebenen Markierungseinheit das Natriumsalz der 3,3-Diphosphono-1,2-propandicarbonsäure als organspezifische "Transportsubstanz" vor. In dem Europäischen Patent 108.253 werden Tc-99m-Tri- und Tetraphosphonsäuren zur szintigraphischen Darstellung des RES, insbesondere der Leber, beschrieben. Der Tc-99m-Komplex mit Diethylentriaminpentaessigsäure (DTPA) findet zur Diagnostik von Nierenerkrankungen bzw. pathologischen Hirnprozessen Anwendung.

Zur Markierung bestimmter Substanzen mit Technetium-99m und zur Herstellung von für den klinischen Routinebedarf geeigneten Testkits wurden spezielle Verfahren entwickelt und beschrieben. Zur Präparierung von Markierungsbestecken für biologisch interessante Makromoleküle, insbesondere Porphyrine, Dextrane, Cytochrome und Myoglobin, wird eine Methode beschrieben (G.D. Zanelli, D. Ellison, M.P. Barrowcliffe, Nucl. Med. Commun. 8, 199-206, 1987), bei der die zu markierende Substanz zusammen mit p-Aminobenzoesäure und einer salzsauren $SnCl_2$-Lösung lyophilisiert wird. Zur Rekonstitution und Markierung dieses Kits gibt man Tc-99m-Generatoreluat, das vorher mit genügend Pufferlösung, z.B. Citrat-Natriumchlorid-Puffer pH 9,5, verdünnt wurde, hinzu. Für säureempfindliche Substanzen ist diese Methode jedoch nicht geeignet.

Bei einem anderen Verfahren (E.K.J. Pauwels, R.I.J. Feitsma, Patentanmeldung Int. Publication No. WO 86/03010) wird durch vierstündiges Erhitzen in stark salzsaurer Lösung auf 140 °C Tc-99m-Pertechnetat zuerst reduziert und an einer Verbindung, die eine Aminogruppe enthält, z.B. Dimethylformamid, gebunden. Das reaktionsfähige Tc-99m-markierte Zwischenprodukt, das als schwerlösliche kristalline Substanz ausfällt, wird in einer Pufferlösung, z.B. Natriumcarbonatlösung, durch einstündige Inkubation bei Raumtemperatur mit der zu markierenden Verbindung umgesetzt. Die Methode arbeitet zwar zinnfrei, ist aber wegen der aufwendigen Verfahrensschritte für die Routineanwendung kaum geeignet.

Zur Markierung von Proteinen, insbesondere Antikörpern, kennt man zwei verschiedene Wege. Bei der direkten Methode wird das reduzierte Technetium-99m durch Donorgruppen (Amino-, Amid-, Thiol- etc.) des Proteins gebunden.

Solche Verfahren sind in dem Europäischen Patent 5638 und dem US-Patent 4.47B.015 beschrieben. Dort werden Zinn-II-Salze im Überschuß zur gleichzeitigen reduktiven Spaltung von Disulfid-Brücken und zur Reduktion des zugesetzten Tc- 99m-Pertechnetats benutzt. Im allgemeinen benötigt man zur Spaltung

der -S-S-Bindung längere Inkubationszeiten (24 Stunden), wobei F(ab′)$_2$-Fragmente partiell zu F(ab′)-Fragmenten gespalten werden. Neuere Literaturangaben (z.B. Journal of Nuclear Medicine 27 (1986), Seite 685 - 93 und 1315 - 20 sowie International Journal of Nuclear Medicine Biology 12 (1985) Seiten 3 - 8) zeigen, daß das Verhältnis der beiden Fragmente abhängig ist von der "Bezinnungsreaktion" und daß sich das Verhältnis der beiden Komponenten nach der Tc-99m-Markierung nicht mehr nennenswert ändert, wobei die Hauptkomponente Tc-99m-markiertes F(ab′) ist. In allen Fällen mußte das markierte F(ab′)-Fragment nachgereinigt werden, da trotz mindestens 30-minütiger Reaktionszeit keine quantitative Umsetzung des Pertechnetats erreicht wurde.

Bei einer schnellen chemischen Methode zur Tc-99m-Markierung humaner Plasmaproteine (D.W. Wong, F. Mishkin, T. Lee, J. Nucl. Med. 20, 967 - 72, 1979) wird Pertechnetat zuerst in saurer Lösung durch Zinn-II-Ionen reduziert und das reduzierte Technetium dann mit dem Protein umgesetzt.

Unter Zuhilfenahme von bifunktionalen Komplexbildnern läßt sich eine stabile Markierung von Substanzen mit Radioisotopen erreichen.

In dem US-Patent 4.479.930 Werden die cyclischen Anhydride von DTPA und EDTA als Chelatisierungsmittel nicht nur für In-111 und Ga-67, sondern auch für Tc-99m angeführt. In dem europäischen Patent 35765 wird die Verwendung von Deferoxamin als Komplexierungsagens für Technetium-99m an Proteine erwähnt. In der internationalen Patentanmeldung WO 85/3063 werden die partiell reduzierten Disulfid-Brücken im Antikörper mit dem Natriumsalz des Tetrachlornitridotechnetats, das durch Reaktion von Pertechnetat mit Natriumazid vorher präpariert werden muß, umgesetzt. In der europäischen Patentanmeldung 194853 benutzt man ebenfalls durch Reduktion in Antikörperfragmenten erzeugte freie Thiolgruppen zur Bindung von [(7-Maleimidoheptyl)imino-bis(ethylennitrilo)]tetraessigsäure als Chelatkomplex. Die Kupplung des Komplexes an den Antikörper erfolgt über die Reaktion der SH-Gruppen mit der Doppelbindung im Maleinimidteil der Komplexverbindung, während das radioaktive Metallion über die Nitrilodiessigsäure-Reste komplexiert wird.

Metallothionein, ein metallbindendes Protein mit einem Molekulargewicht von 6000 und einem hohen Cysteinanteil im Molekül wurde als Komplexbildner in Antikörper eingeführt (G.L. Tolman, R.J. Hadjian, M.M. Morelock et al., J. Nucl. Med. 25, 20, 1984). Durch Austausch mit Tc-99m-Glucoheptonat ließ sich das Antikörper-Metallothionein-Konjugat mit Technetium markieren. Der Austausch blieb jedoch unvollständig, so daß eine Nachreinigung erforderlich war. Mehrere Bisthiosemicarbazon-Liganden wurden ebenfalls als bifunktionelle Chelatbildner beschrieben (Y. Arano, A. Yokoyama, H. Magat et al., Int. J. Nucl. Med. Biol. 12, 425 - 30, 1986). p-Carboxyethylphenylglyoxal-di(N-methylthiosemicarbazon) wurde mit humanem Serumalbumin konjugiert. Der Tc-99m-markierte 1:1-Komplex zeigte eine gewisse Instabilität, während Komplexe mit einem höheren Verhältnis als 1:1 eine vermehrte Leberspeicherung aufwiesen. Die Kopplung eines Diamid-dimercaptid-$N_2S_2$-Liganden an Proteine (A.R. Fritzberg, S. Kasina, J.M. Reno et al., J. Nucl. Med. 27, 957 - 958, 1906) erfolgt über eine zusätzliche funktionelle Gruppe. So wurde z.B. 4,5-Di(S-ethylcarbonylmercaptoacetamid)pentanoyl-N-hydroxysuccinimid mit einem anti-Melanomantikpörper umgesetzt. Das entstandene Konjugat wurde bei pH 8 und 50 °C mit Tc-99m-Tartratlösung inkubiert. Nach einer Stunde waren 78 % des Technetiums vom Tartrat auf den Antikörper übertragen.

Um Technetium-99m diagnostisch breit nutzen zu können, ist es notwendig, dieses Nuklid in das zu untersuchende Organ selektiv zu transportieren. Aus anderen Organen oder Organsystemen sollte das Technetium-99m wieder schnell eliminiert oder überhaupt nicht hingebracht werden, um jede unnötige Strahlenbelastung für den Patienten zu vermeiden. Zu diesem Zweck werden bisher vorwiegend Substanzen eingesetzt, die direkt mit Technetium-99m markierbar sind und eine hohe Organspezifität aufweisen. Darüber hinaus gibt es jedoch eine Reihe von Substanzen, die zwar eine hohe Organspezifität aufweisen, aber nicht direkt markierbar sind. Dies können Proteine (Fibrinogen, Humanserumalbumin), Enzyme (Streptokinase, Lactatdehydrodgenase), Zucker (Dextran, Glucose) oder auch Polymere sein. Dazu gehören ebenfalls niedermolekulare Substanzen wie etwa Fettsäuren, die durch den hohen Energiebedarf des Herzens sich im Myocardgewebe anreichern. Um diese Substanzen markieren zu können, werden sie mit Komplexbildnern gekoppelt, die ihrerseits Technetium-99m fest binden können.

Als geeignete Komplexbildner für die Komplexierung von Technetium- und Rheniumisotopen sind makrozyklische Amine, u.a. auch Cyclame, bekannt. Die Komplexierungsausbeute liegt für den Technetium-Cyclam Komplex bei 99 % unter geeigneten Bedingungen. Einzelheiten über Technetium-Aminkomplexe sind in D.E. Troutner, J. Simon, A.R. Ketring, W.A. Volkert, R.A. Holmes, J. Nucl. Med. 21 (1980), 443 oder S.A. Zuckman, G.M. Freeman, D.E. Troutner, W.A. Volkert, R.A. Holmes, D.G. van der Keer, E.K. Barefiled, Inorg. Ch. 20 (1981), 3386 oder J. Simon, D. Troutner, W.A. Volkert, R.A. Holmes, Radiochem. Radioanal. Lett. 47 (1981), 111 erwähnt. Auch substituierte Cyclame sind sowohl am 1-Stickstoff, als auch am 6-Kohlenstoff substituiert, bekannt (A.R. Ketring, D.E. Troutner et al., Int. J. Nucl. Med. Biol. 11 (1984), 113 oder J. Simon. Diss. Abstr. Int. B42 (1981), 645 oder M. Struder, T.A. Kaden, Helv. Chim. Acta 69 (1986),

2067 oder E. Kimura, R. Machida, M. Kodama, J. Am. Chem. Soc. 106 (1984), 5497).

Alle bisherigen Versuche, Amin- und auch andere Liganden an Proteine zu konjugieren (s. Fritzberg et al., J. Nucl. Med. 27 (1986), 957 oder Tolman et al., J. Nucl. Med. 25 (1984), 20 oder Arano et al., Int. J. Nucl. Med. Biol. 12 (1986) 425) führten zu Produkten, die die hohen in vivo Stabilitätsansprüche nicht oder nur teilweise erfüllten.

In der DE-A 3728600 wurden N-alkyl bzw. N-aryl und C-substituierte Cyclame beschrieben, welche sich ebenfalls als Komplexierungsmittel für Radionuklide eignen.

Es wurden nun 1,4,7,10-Tetraazacyclotridecane gefunden, welche bevorzugt an den vier Stickstoffatomen mit Alkylphosphonaten und/oder -acetaten und/oder -sulfonaten und/oder -phenolaten und/oder -catecholaten und/oder -salicylaten substituiert sind und in 12-Positionen einen Rest tragen, der zur Bindung an eine zu markierende Substanz befähigt ist. Die erfindungsgemäßen 1,4,7,10-Tetraazacyclotridecane sind hervorragend zur Komplexierung von Radionukliden geeignet. Bevorzugt werden mit diesen Verbindungen die Radionuklide $^{111}In$, $^{68}Ga$, $^{67}Ga$, $^{90}Y$, $^{153}Sm$, $^{52}Fe$, $^{99m}Tc$, $^{153}Gd$, $^{186}Re$ oder $^{188}Re$ aber auch Metalle, die als NMR-Kontrastmittel eingesetzt werden können, wie Gd, Fe oder Mn, komplexiert.

Unter den "Substanzen" die mit Hilfe der erfindungsgemäßen Cyclotridecane markierbar sind, werden in erster Linie solche Verbindungen verstanden, die in der medizinischen Diagnostik als "Transportsubstanzen" eingesetzt werden können, also meist Verbindungen, die eine hohe Organspezifität aufweisen wie Antikörper, Antikörperfragmente wie $F(ab')_2$- oder $F(ab')$-Fragmente, Proteine wie Fibrinogen, Humanserumalbumin, Steroide, Lipide, Enzyme wie Streptokinase, Lactatdehydrogenase, Zucker wie Dextran, Glucose oder auch Polymere. Andererseits können aber auch generell solche Substanzen mit Hilfe der erfindungsgemäßen Cyclotridecane markiert werden, die mit der funktionellen Gruppe in 12-Position an der Seitenkette des makrocyclischen Amins unter Ausbildung einer chemischen Bindung reagieren. Gedacht ist hierbei an die "Überwachung" von chemischen Substanzen in Produktionsanlagen, die Bestimmung ihrer Konzentration, Strömungsgeschwindigkeit, Verweilzeit etc.

Die Erfindung betrifft somit 1,4,7,10-Tetraazacyclotridecane der Formel I

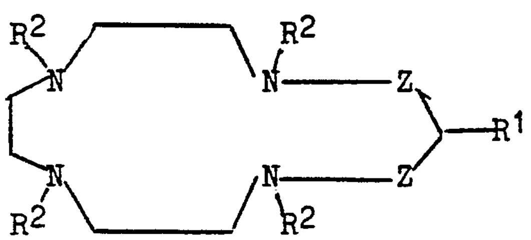

(I)

worin

Z $CH_2$ oder CO bedeutet und

$R^1$ eine Gruppe der Formel $-X-NH_2$, $-X-NO_2$, -X-NUS, -X-COOH, -X-OH, $-X-N_2^+$ oder -X-COCl oder eine Gruppe der Formel X-Hal bedeutet, wobei Hal, Fluor, Chlor, Brom oder Jod bedeutet und X eine Alkylengruppe mit 1 bis 40 C-Atomen, eine ortho-, meta- oder para-Phenylen-, oder eine ortho-, meta- oder para-Phenyl-$C_1$-$C_2$-alkylengruppen bedeutet und

$R^2$ H oder $-(CH_2)_n-R^3$ bedeutet wobei

n 1 oder 2 ist und

$R^3$ -COOH, $-PO_3H_2$, $-SO_3H$

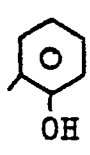 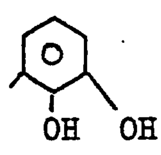 oder 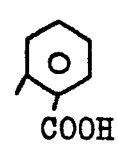

bedeutet

und wobei die vier $R^2$-Reste verschieden sind oder 2, 3 oder alle $R^2$-Reste identisch sind.

Insbesondere betrifft die Erfindung 1,4,7,10-Tetraazacyclotridecane der Formel I wie oben definiert, wobei jedoch mindestens eine der nachfolgenden Bedingungen erfüllt ist:

Z bedeutet $CH_2$

X bedeutet eine Alkylengruppe mit 1 bis 20 C-Atomen oder

X bedeutet eine para-Phenylengruppe oder
X bedeutet eine para-Phenylmethylengruppe
Hal bedeutet Chlor, Brom oder Jod
n ist 1
$R^3$ bedeutet -COOH, $-PO_3H_2$ oder $-SO_3H$
3 oder alle $R^2$-Reste sind identisch.

Ganz besonders bevorzugt sind 1,4,7,10-Tetraazacyclotridecane der Formel I wie oben definiert wobei jedoch mindestens eine der nachfolgenden Bedingungen erfüllt ist:
Z bedeutet $CH_2$
X bedeutet eine Alkylengruppe mit 1 bis 8 C-Atomen oder
X bedeutet eine para-Phenylengruppe oder
X bedeutet eine para-Phenylmethylengruppe
Hal bedeutet Chlor, Brom oder Jod
n ist 1
$R^3$ bedeutet -COOH oder $PO_3H_2$
alle $R^2$-Reste sind identisch.

Bevorzugt ist in den erfindungsgemäßen Verbindungen immer ein $R^2$-Substituent von H verschieden.

Ein weiterer Gegenstand der Erfindung ist ein Verfahren zur Herstellung der erfindungsgemäßen 1,4,7,10-Tetraazacyclotridecane, wobei man zunächst einen Malonsäuredialkylester der Formel II

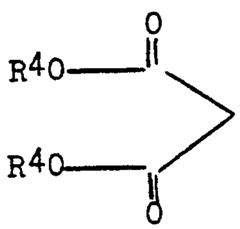

(II)

worin $R^4$ $C_1$-$C_4$-Alkyl bedeutet,
mit einer Verbindung der Formel III
A - $R^1$ (III)
worin $R^1$ die oben zu Formel I angegebenen Bedeutungen hat und A Chlor oder Brom bedeutet, umsetzt
und anschließend die erhaltene Verbindung der Formel IV

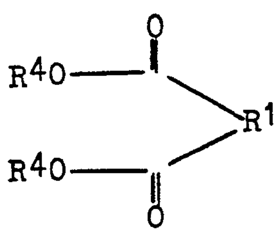

(IV)

mit Triethylentetraamin der Formel V

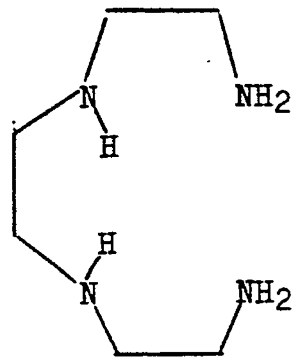

(V)

zu Verbindungen der Formel VI

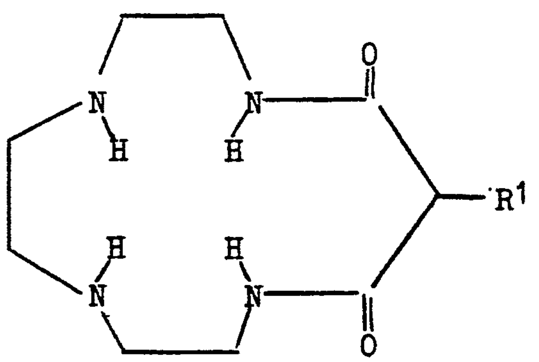

(VI)

umsetzt, die CO-gruppen in 11,13-Position zu $CH_2$-Gruppen reduziert oder nicht reduziert und anschließend die erhaltene Verbindung der Formel VII

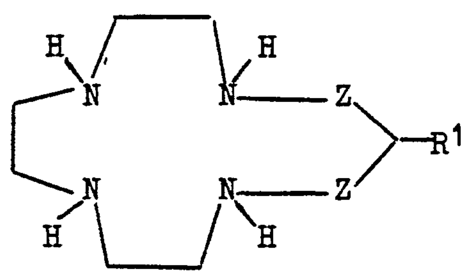

(VII)

mit Verbindungen der Formel VIII

A - $R^2$

worin A Chlor oder Brom bedeutet und $R^2$ die oben zu Formel I angegebenen Bedeutungen hat, zu Verbindungen der Formel I umsetzt.

Weiterhin betrifft die Erfindung die Verwendung der erfindungsgemäßen Cyclotridecane zur Komplexierung von Radionukliden sowie die Verwendung dieser Komplexe zur Markierung von Substanzen. Ebenso betrifft die Erfindung die Verwendung dieser markierten Substanzen, insbesondere in der medizinischen Diagnostik.

Alkylgruppen mit mehr als 2 C-Atomen können sowohl geradkettig als auch verzweigt sein. Bei der Alkylengruppe X handelt es sich bevorzugt um geradkettige Alkylene mit bis zu 40 C-Atomen, bevorzugt bis zu 20, besonders bevorzugt bis zu 8 C-Atomen.

Die erfindungsgemäßen Cyclotridecane bsitzen in 12-Position einen Substituenten mit funktioneller Gruppe. Mit Hilfe dieser funktionellen Gruppe werden die erfindungsgemäßen Cyclotridecane an die zu markierende Substanz gebunden. Gegebenenfalls nach entsprechender Reinigung des Konjugats (z.B. durch Ultrafiltration oder Dialyse bei Proteinen oder Polymeren, durch Säulenchromatographie bei niedermolekularen Substanzen wie Steroiden oder Lipiden) wird das gewünschte Radionuklid zwecks Komplexierung hinzugegeben. Beispielsweise wird Technetium-99m in der Form von Pertechnetat bzw. Rhenium-186

oder -188 in der Form von Perrhenat und ein geeignetes Reduktionsmittel zur Reduktion des Pertechnetats bzw. Perrhenats in die für die Komplexierung benötigte Oxidationsstufe, in beliebiger Reihenfolge oder gemeinsam zugegeben. Das markierte Substrat wird gegebenenfalls nochmals gereinigt.

Alternativ kann auch zunächst der Radionuklidkomplex des Cyclotridecans hergestellt werden und dann dieser mit der Substanz zum Konjugat umgesetzt werden.

Zur Herstellung der erfindungsgemäßen 1,4,7,10-Tetraazacyclotridecane geht man am besten so vor, daß man substituierte Malonsäurederivate (IV) mit Triethylentetramin (V) umsetzt, sofern gewünscht, die vorhandenen beiden CO -gruppen reduziert und anschließend die gewünschten $R^2$-Gruppen in das Molekül einführt (siehe Fig. 4; Reaktionsschema 1). Die entsprechend substituierten Malonsäurederivate erhält man beispielsweise durch Umsetzung von Malonsäuredialkylester, bevorzugt Malonsäurediethylester mit einer Verbindung der Formel III, A-$R^1$ (III) wobei A Chlor oder Brom bedeutet und $R^1$ die oben zu Formel I angegebenen Bedeutungen hat. Bevorzugt wird die Reaktion in Gegenwart eines Lösungsmittels wie Tetrahydrofuran, unter gleichzeitiger Einwirkung eines protanenabspaltenden Mittels, wie einer Base, Wie Lithiumdiisopropylamid durchgeführt. Die Reaktionstemperatur beträgt -80 bis +20 °C, bevorzugt -70 bis -10 °C. Die Reaktionszeit beträgt 10 Min bis 2 Std., bevorzugt 10 Min bis 1 Std.

Das erhaltene substituierte Malonsäurederivat der Formel IV wird nun im folgenden Reaktionsschritt mit Triethylentetramin (V) umgesetzt. Auch diese Reaktion erfolgt bevorzugt in einem Lösungsmittel wie Methanol, Ethanol, i-Propanol. Die Reaktionstemperatur liegt hierbei zwischen 50 °C und dem Siedepunkt des Reaktionsgemisches, bevorzugt wird bei Siedetemperatur gearbeitet. Die Reaktionszeiten betragen 12 bis 240 Std., bevorzugt 24 bis 170 Std.

Sofern gewünscht können nun die beiden im Molekül befindlichen Ketogruppen zu Methylengruppen reduziert werden. Dazu wird das 11,13-Dion zweckmäßigerweise in einem Lösungsmittel wie THF mit einem Reduktionsmittel wie $LiAlH_4$, oder $BH_3$ versetzt. Die Umsetzung erfolgt bei Temperaturen von 50 °C bis zum Siedepunkt der Reaktionsmischung, bevorzugt in der Siedehitze. Die Reaktionszeiten betragen 5 bis 48 Std., bevorzugt 12 bis 24 Std.

Zur Überführung in die entsprechend $R^2$-substituierten Cyclotridecane versetzt man entweder das 11,13-Dion oder das reduzierte Produkt mit den Verbindungen der Formel VIII. Dies geschieht bevorzugt in Gegenwart eines Lösungsmittels wie Wasser, i-Propanol oder Dimethylformamid bei gleichzeitiger Anwesenheit einer Base wie KOH, NaOH, $Na_2CO_3$ oder $Li_2CO_3$. Die Reaktionstemperatur beträgt 50 °C bis Siedetemperatur des Reaktionsgemischs, bevorzugt 60 - 80 °C. Die Reaktionszeiten betragen 20 Min bis 140 Min, bevorzugt 60 bis 120 Min.

Die erhaltenen Endprodukte können beispielsweise mittels Ionenaustausch-Chromatographie gereinigt werden. Die zur Herstellung der erfindungsgemäßen Verbindungen notwendigen Ausgangssubstanzen sind entweder käuflich erhältlich oder nach literaturbekannten Verfahren leicht darzustellen.

Es ist außerordentliche überraschend, daß die erfindungsgemäßen 13-Ring-Cyclame im Vergleich zu den bekannten, strukturell verwandten 12-Ring und 14-Ring Cyclamen eine höhere oder gar wesentlich höhere Komplexstabilität aufweisen. Dies geht aus Vergleichsversuchen mit 12-Ringcyclamen (DOTA; "Dodecatetraacetate") und 14-Ringcyclamen (TETA; "Tetradecantetraacetat") hervor (s. Fig. 1; als weitere Vergleichssubstanz ist in der Figur 1 DTPA (Diethylentriaminpentaessigsäure) aufgeführt). Bei diesen Vergleichsversuchen wurde $^{111}$In sowohl mit den bekannten 12- und 14-Ring-Cyclamen als auch mit dem erfindungsgemäßen Cyclam aus Beispiel 1 komplexiert und anschließend jeweils der $^{111}$In-Austausch gegen Transferrin - ein bekanntermaßen gutes Komplexierungsmittel - in humanem Serum bei 37 °C untersucht. Während das (die) 14-Ring-Cyclam(e) bereits nach 12 Stunden einen merklichen $^{111}$In-Austausch erkennen ließ (ca. 10 % des zuvor vom 14-Ring-Cyclam komplexgebundenen $^{111}$In wurde nach dieser Zeit freigesetzt bzw. von Transferrin komplexiert), zeigte sich bei dem 12-Ring-Cyclam und bei dem erfindungsgemäßen 13-Ring-Cyclam nach dieser Zeit nur ein geringer $^{111}$In-Austausch (ca. 0,3% für das 12-Ring-Cyclam und $<$ 0,1 % für das erfindungsgemäße 13-Ring-Cyclam). Die Überlegenheit der erfindungsgemäßen 13-Ring-Cyclame gegenüber den 12-Ring-Cyclamen und erst recht gegenüber den 14-Ring-Cyclamen wird nach längerer Einwirkzeit deutlich. So wurden naoh 120 Stunden im Fall des 14-Ring-Cyclams schon über 30 % des $^{111}$In ausgetauscht, im Fall des 12-Ring-Cyclams waren es immerhin ca. 4 %, Während nur ca. 1 % des $^{111}$In aus dem erfindungsgemäßen 13-Ring-Cyclam ausgetauscht wurden (s. Fig. 1). Selbst bei noch längeren Reaktionszeiten nimmt die $^{111}$In-Austauschrate im Fall der erfindungsgemäßen 13-Ring-Cyclame nicht mehr wesentlich zu, während für die 12-Ring- und 14-Ring-Cyclame ein weiterer Anstieg des $^{111}$In-Austauschs zu beobachten ist.

In Fig. 2 ist die "$^{111}$In-Komplexstabilität der erfindungsgemäßen Cyclotridecane im Vergleich zu Diethylentriamin-paranitrobenzyl-tetraessigsäure aufgeführt.

Im folgenden wird die Erfindung anhand von Beispielen näher erläutert und in den Patentansprüchen definiert.

**Beispiel 1**

Darstellung des 12-substituierten Cyclams 12-(p-Nitrobenzyl)-1,4,7,10-tetraacetat-1,4,7,10-tetraazacyclotridecan

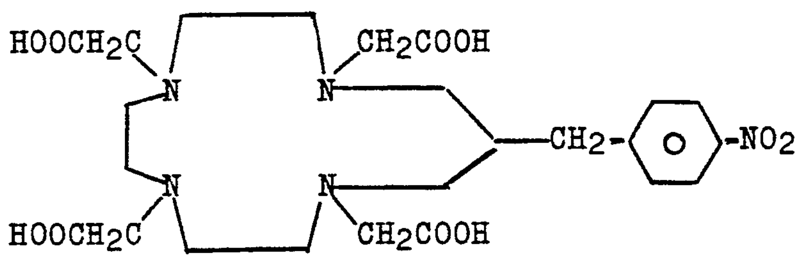

a) Darstellung von Mono-p-nitrobenzylmalonsäurediethylester

**Schema:**

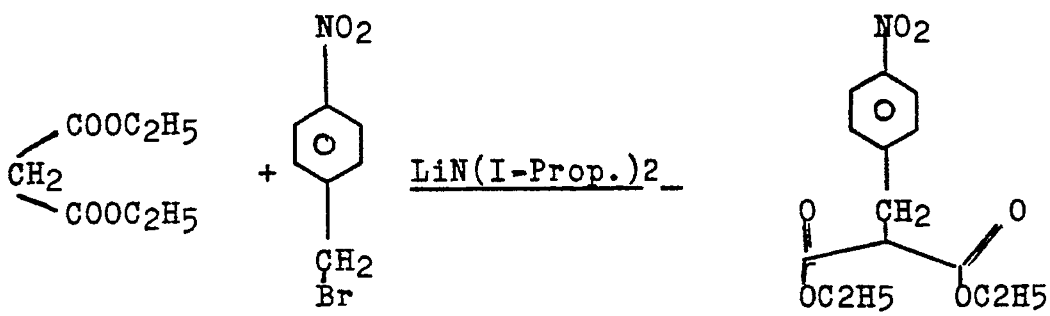


**Ansatz:**

0,181 mol
58,0 g Malonsäurediethylester (0,362 mol)
39,1 g p-Nitrobenzylbromid (0,181 mol)
21,4 g Lithiumdiisopropylamid (0,2 mol)
**Ausbeute:** 65 % (34,7 g)

Unter Stickstoff wird die Lithiumbase mit 210 ml wasserfreiem Tetrahydrofuran in einen gut getrockneten, mit Stickstoff ausgeblasenen Dreihalskolben mit Rührer gegeben. Mit einem Tropftrichter wird nun bei Raumtemperatur der in 100 ml THF gelöste Malonester innerhalb 40 Min. zugetropft. Man erwärmt leicht, um nicht gelöstes Lithiumdiisopropylamid in Lösung zu bringen. Die nun orange-braune Reaktionslösung kühl man mit Trockeneis/Isopropanol auf -62° C ab.

Das in THF gelöste p-Nitrobenzylbromid wird innerhalb 40 Min. unter starkem Rühren zugetropft. Man rührt eine Stunde bei -62° C und filtriert dann den gebildeten Festkörper (Smp. 235° C) kalt ab. Das Filtrat wird am Rotavap eingedampft. Der Rückstand wird in ca. 300 ml Ethanol bei ca. 65° C in lösung gebracht. Dabei kommt ein nichtlöslicher Festkörper zum Vorschein (Smp. 160° C,· Disubstituiertes Nebenprodukt), den man warm abnutscht. Man läßt im Kühlschrank auskristallisieren. Als Produkt erhält man 34,7 g schwach gelbliche Kristalle mit Smp. 57-58° C.

**Identifikation:**

Schmelzpunkt 58° C
IR:
1740 $cm^{-1}$ C=O
1530 $cm^{-1}$ as$^{NO}$2
1350 $cm^{-1}$ sym$^{NO}$2
$^1$H-NMR: ($CDCl_3$)
8,1 d 2H Bz

7,4 d 2H Bz
4,1 q 4H $-CH_2-CH_3$
3,6 t 1H CH
3,3 d 1H $Ar-CH_2-CH$
1,2 t 6H $-CH_2-CH_3$

| Elementaranalyse: | ber. | gef. |
|---|---|---|
| C | 56,95 | 56,8 |
| H | 5,80 | 5,8 |
| N | 4,74 | 5,0 |

DC:
$CHCl_3$ / MeOH / $NH_3$ (2:2:1)
Rf: 0,89
EtOH
Rf:
Monosubstituiertes Produkt 0,93
Disubstituiertes Produkt 0

b) Darstellung von 12-(p-Nitrobenzyl)-1,4,7,10-tetraazacyclotridecan-11,13-dion

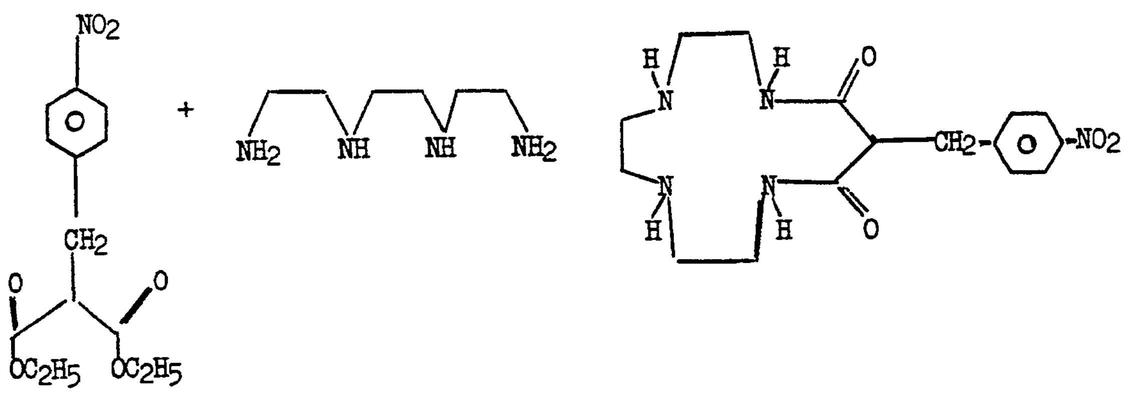

Ansatz:
0,067 mol
20,0 g Mono-p-Nitrobenzylmalonsäurediethylester aus Beispiel 1a (0,067 mol)
10,0 g Triethylentetramin (0,068 mol)
200 ml Ethanol

Die Edukte werden in Ethanol gelöst und anschließend während 7 Tagen im Rückfluß gekocht.

Innerhalb 7 Tagen bildet sich ein Niederschlag (=> Produkt), der nach Abkühlen der Reaktionslösung und anschließender Filtration gefaßt werden kann. Nach waschen mit Aceton erhält man ein feines, leicht gelbliches Pulver.

Ausbeute: 25 % der Theorie (5,9 g)
Identifikation:
Schmelzpunkt : Zersetzung ab ca. 200 °C
DC-System: MeOH / $CHCl_3$ / $NH_3$ (2:2:1)
Rf-Wert = 0,9

| Elementaranalyse: | ber. | gef. |
|---|---|---|
| C | 54,36 % | 54,23 % |
| H | 6,71 % | 6,80 % |
| N | 19,81 % | 19,89 % |
| $H_2O$ | 1,17 % | 1,17 % |

NMR-Spektren

$^1H$ (400 MHz) ($CDCl_3$)

8,144 – 8,122 d 2H

| | | |
|---|---|---|
| | 7,416<br>7,394 | d 2H |
| | 2,6 - 3,6 | m 15H |
| $^{13}C$ (101 MHz)<br>($CDCl_3$) | 169,3 | C=O |
| | 146,9<br>146,6 | 2 aromat. C |
| | 129,8<br>123,8 | 2 aromat. CH |
| | 57,3 | aliph. CH |
| | 47,79<br>46,87<br>39,59<br>34,92 | 4 aliph. $CH_2$ |

c) Darstellung von 12-(p-Nitrobenzyl)-1,4,7,10-tetraazacyclotridecan

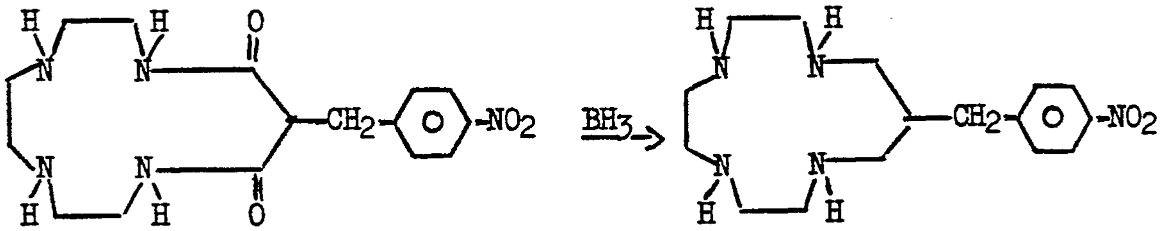

**Ansatz:**

0,0212 mol

7,4 g 12-(p-Nitrobenzyl-1,4,7,10-tetraazacyclotridecane-11,13-dione aus Beispiel 1b (0,0212 mol)

220 ml $BH_3$ in THF (0,220 mol)

100 ml THF trocken

Das Edukt wird in 100 ml trockenem THF suspendiert und anschließend $BH_3$ in THF langsam zugegeben. (=> $H_2$-Bildung). Die Reaktionslösung wird auf Rückfluß erhitzt, wobei das Amid langsam in Lösung geht.

Reaktionszeit: 24 h unter Rückfluß.

Zur heißen Reaktionslösung wird in sehr kleinen Portionen 200 ml MeOH zugegeben um das überschüßige $BH_3$ zu entfernen.

Anschließend wird die Reaktionslösung 3x zur Trockne eingedampft und wieder mit MeOH aufgenommen (entfernen der Borester). Zur eingedampften Reaktionslösung werden 300 ml konz. HCl zugegeben und für 3 h auf Rückfluß erhitzt.

Nach Einengen der Reaktionslösung auf ca. 1/3 des Volumens, 200 ml MeOH zugeben und abkühlen lassen. Es bildet sich ein weißer Niederschlag, der abfiltriert und mit kaltem MeOH gewaschen werden kann (Produkt).

**Ausbeute:** 67 % der Theorie (6,9 g)

**Identifikation:**

Mit $CHCl_3$/ MeOH / $NH_3$ 2/2/1 : flammenförmiger Fleck (UV positiv) mit RF = 0

| Elementaranalyse: | ber. | gef. |
|---|---|---|
| C | 39,22 % | 39,3 % |
| H | 6,91 % | 7,0 % |
| N | 14,29 % | 14,1 % |
| $H_2O$ | 4,64 % | 4,6 % |
| Cl | 28,94 % | 29,1 % |

NMR-Spektren

| | | | |
|---|---|---|---|
| $^1H$ (400 MHz) ($D_2O$) | 7,575 – 7,554 | d | 2H aromat. |
| | 8,166 – 8,144 | d | 2H aromat. |
| | 2,806 – 2,813 – 2,823 | t | 1H $-CH_2-\underline{CH}$ |
| | 3,053 – 3,035 | d | 2H $-\underline{CH_2}-CH$ |
| | ~ 3,5 | m | 16H $-\underline{CH_2}-$ |

| $^{13}C$ (101 MHz) ($D_2O$) | | |
|---|---|---|
| | 149,36<br>148,26 | 2 aromat. C |
| | 133,06<br>126,84 | 2 aromat. CH |
| | 50,71<br>47,74<br>47,44<br>46,43<br>38,52 | 5 aliph. $CH_2$ |
| | 37,92 | 1 aliph. CH |

d) Darstellung des 12-(p-Nitrobenzyl)-1,4,7,10-tetraacetat-1,4,7,10-tetraazacyclotridecans

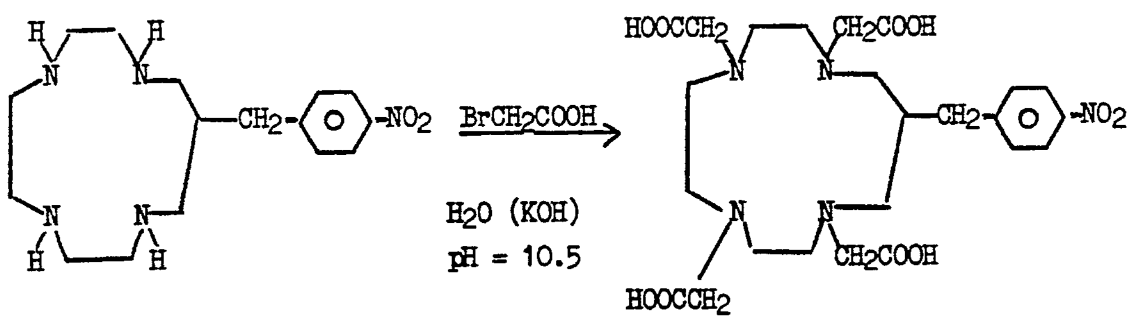


**Ansatz:**

2,0 g 12-(p-Nitrobenzyl)tetraazacyclotridecan aus Beispiel 1c ($4,28x10^{-3}$ mol) gelöst in 30 ml $H_2O$ dest. mit KOH 6M auf pH 10,5

3,0 g Bromessigsäure ($2,14x10^{-2}$ mol) gelöst in 20 ml $H_2O$ dest.

12-(p-Nitrobenzyl)-tetraazacyclotridecan-hydrochlorid wurde in 30 ml $H_2O$ dest. gelöst und mittels KOH 6M auf pH 10,5 gestellt.

Die Reaktionslösung wurde auf 75 °C erhitzt und die in 20 ml $H_2O$ dest. gelöste Bromessigsäure während ca. 20 Min zugetropft.

Der pH wurde mittels eines pH-Staten und KOH 6M konstant gehalten. Nachrührzeit 2 h, anschließend Reaktionslösung zur Trockne eingedampft (Rückstand ca. 9,5 g).

**Aufarbeitung:**

Die Reinigung des Produkts von Salzen und Nebenprodukten erfolgte mittels Ionenaustausch-Chromatographie.

Ionenaustauscher: Dowex 1 x 4 20-50 mesh, Formiatform
Säule: 2,5 x 36 cm
Detektion: UV 275 nm

Der Festkörper wurde in 30 ml $H_2O$ dest. aufgenommen und filtriert. Nur ca. 15 ml auf die Säule aufgetragen und mit $H_2O$ dest. eluiert.

1. Peak: anorganisches Salz
2. Peak: Nebenprodukt (Tri acetat FAB-MS: $M^+H$ = 496)

Das Produkt wurde mit 0,1 M Ameisensäure vom Ionenaustauscher eluiert (=> 3. Peak FAB-MS: $M^+H$ = 554).

| Elementaranalyse: $C_{24}H_{35}N_5O_{10}$ • 0,64 HBr • 0,9 $H_2O$ | | |
|---|---|---|
| | ber. | gef. |
| C | 48,08 | 47,6 |
| H | 6,32 | 6,6 |
| $H_2O$ | 2,7 | 2,7 |
| N | 11,7 | 11,67 |
| Br | 8,54 | 8,6 |

NMR-Spektren

$^1H$ ($D_2O$, 400 MHz)

| | | | |
|---|---|---|---|
| 8,21 – 8,185 | d | 2H aromat. |
| 7,497 – 7,475 | d | 2H aromat. |
| 2,5 | t | 1H aliphat. |
| 2,7 - 3,9 | m | 26H aliphat. |

| | | |
|---|---|---|
| $^{13}C$ ($D_2O$, 101 MHz) | 179,7 | -COOH |
| | 178,2 | -COOH |
| | 149,735<br>149,424 | 2 aromat. C |
| | 133,016<br>126,845 | 2 aromat. CH |
| | 37,285 - 63,555 | 7 aliphat. $CH_2$<br>1 aliphat. CH |

**Beispiel 2**

Darstellung von 12-(p-Nitrobenzyl)-1,4,7,10-tetramethylphosphonat-1,4,7,10-tetraazacyclotridecan

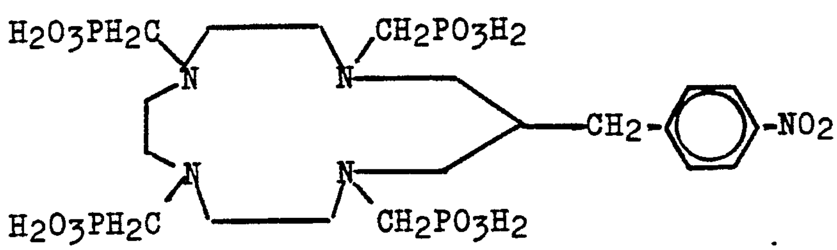

1,4 g 12-(p-Nitrobenzyl)-1,4,7,10-tetraazacyclotridecan hydrochlorid (aus Beispiel 1c) wurde mit 2 g $H_3PO_3$ in 20 ml $H_2O$ und 20 ml HCl konz. auf nahe Siedetemperatur gebracht. Über 1 Std. wurden 15 ml $CH_2O$ (35 % in $H_2O$) zugetropft. Es wurde 2 Std. am Rückfluß gekocht und nochmals 15 ml $CH_2O$ zugegeben. Dann über Nach am Rückfluß gekocht. Das Reaktionsgemisch wurde dann eingedampft und mit absolutem EtOH (50 ml) ein gelber Festkörper extrahiert. Die EtOH-Lösung wurde auf 10 ml reduziert; nach Zugabe von Isopropanol fielen noch einmal 0,25 g Festkörper an. Die vereinigten Festkörper wurden in $H_2O$ gelöst und via Anionenaustauscher (Dowex 1 x 4) mit einem Gradienten 0,1-0,5 M HCOOH eluiert. 3 Fraktionen wurden bei 270 nm detektiert wobei die zweite Fraktion dem trisubstituierten und die dritte Fraktion dem tetrasubstituierten Produkt entspricht.

$^1H$-NMR, 360 MHZ:

3. Fraktion

7,5 d 2 aromat. Protonen
8,2 d 2 aromat. Protonen
2,6-3,8 m 27 Protonen
2. Fraktion
7,5 d 2 aromat. Protonen
8,2 d 2 aromat. Protonen
2,6-3,8 m 25 Protonen

**Beispiel 3**

Synthese eines $^{153}Gd^{3+}$-Komplexes mit der Verbindung aus Beispiel 1

40 mg der Substanz aus Beispiel 1 und 100 µ Ci $^{153}GdCl_3$ werden in 10 ml 0,1 M Natriumacetat-Puffer (pH = 5) über Nacht bei 80 °C gehalten. Nach dieser Inkubationszeit wurde kein freies $Gd^{3+}$ mehr gefunden.

Fig. 3 zeigt die Stabilität dieses Gd-Komplexes in Magensaft bei einem pH-Wert von 1,1. Die Trennung von freiem und noch gebundenem $^{153}Gd^{3+}$ erfolgte über einen Kationenaustauscher.

**Ansprüche**

1. 1,4,7,10-Tetraazacyclotridecane der Formel I

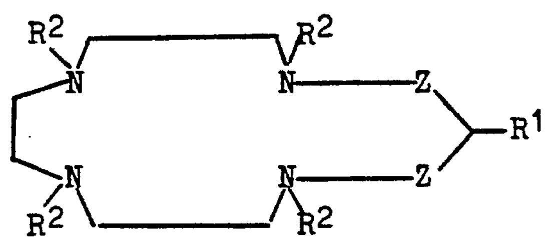

(I)

worin
Z $CH_2$ oder CO bedeutet und
$R^1$ eine Gruppe der Formel $-X-NH_2$, $-X-NO_2$, -X-NCS, -X-COOH, -X-OH, $-X-N_2^+$ oder -X-COCl oder eine Gruppe der Formel X-Hal bedeutet, wobei Hal, Fluor, Chlor, Brom oder Jod bedeutet und X eine Alkylengruppe mit 1 bis 40 C-Atomen, eine ortho-, meta- oder para-Phenylen-, oder eine ortho-, meta- oder para-Phenyl-$C_1$-$C_2$-alkylengruppe bedeutet und
$R^2$ Wasserstoff oder $-(CH_2)_n-R^3$ bedeutet, wobei
n 1 oder 2 ist und
$R^3$ -COOH, $-PO_3H_2$, $-SO_3H$,

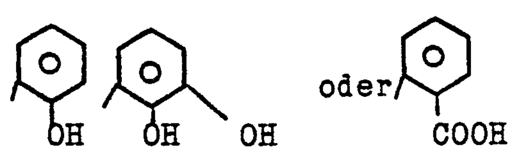

bedeutet
und wobei die vier $R^2$-Reste verschieden sind oder 2, 3
oder alle $R^2$-Reste identisch sind.

2. 1,4,7,10-Tetraazacyclotridecane gemäß Formel I nach Anspruch 1, wobei mindestens eine der nachfolgenden Bedingungen erfüllt ist:
Z bedeutet $CH_2$
X bedeutet eine Alkylengruppe mit 1 bis 20 C-Atomen oder

X bedeutet eine para-Phenylengruppe oder
X bedeutet eine para-Phenylmethylengruppe
Hal bedeutet Chlor, Brom oder Jod
n ist 1
$R^3$ bedeutet -COOH, $-PO_3H_2$ oder $-SO_3H$,
3 oder alle $R^2$-Reste sind identisch.

3. 1,4,7,10-Tetraazacyclotridecane gemäß Formel I nach Anspruch 1 oder 2, wobei mindestens eine der nachfolgenden Bedingungen erfüllt ist:
Z bedeutet $CH_2$
X bedeutet eine para-Phenylengruppe oder
X bedeutet eine para-Phenylmethylengruppe oder
X bedeutet eine Alkylengruppe mit 1 bis 8 C-Atomen
Hal bedeutet Chlor, Brom oder Jod
n ist 1
$R^3$ bedeutet -COOH, oder $-PO_3H_2$
alle $R^2$-Reste sind identisch.

4. 1,4,7,10-Tetraazacyclotridecane nach einem oder mehreren der Ansprüche 1 bis 3, dadurch gekennzeichnet, daß mindestens ein $R^2$-Substituent von Wasserstoff verschieden ist.

5. Verfahren zur Herstellung von 1,4,7,10-Tetraazacyclotridecanen der Formel I gemäß Anspruch 1, dadurch gekennzeichneit, daß ein Malonsäuredialkylester der Formel II

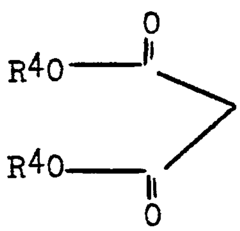

(II)

worin $R^4$ $C_1$-$C_3$-Alkyl bedeutet,
mit einer Verbindung der Formel III
$A - R^1$ (III)
worin $R^1$ die in Anspruch 1 zu Formel I angegebenen Bedeutungen hat und A Chlor oder Brom bedeutet, umsetzt
und anschließend die erhaltene Verbindung der Formel IV

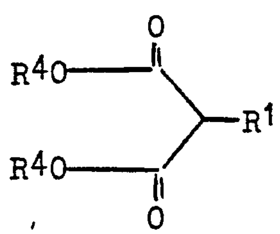

(IV)

mit Triethylentetraamin der Formel V

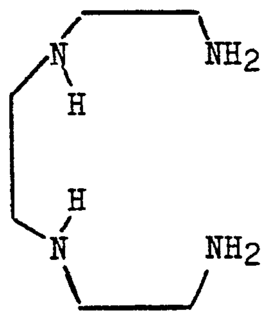

(V)

zu einer Verbindung der Formel VI

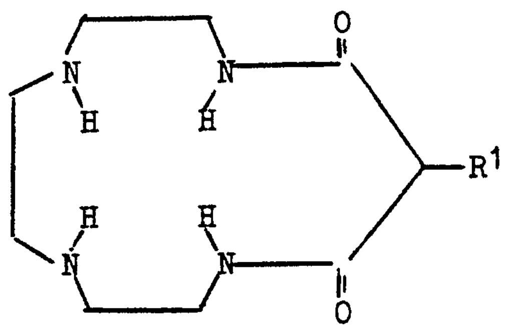

(VI)

umsetzt,
die CO- gruppen in 11,13-Position zu $CH_2$-Gruppen reduziert oder nicht reduziert und anschließend die erhaltene Verbindung der Formel VII

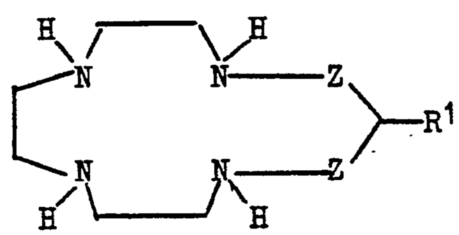

(VII)

mit Verbindungen der Formel VIII
A - $R^2$
worin A Chlor oder Brom bedeutet und $R^2$ die in Anspruch 1 zu Formel I angegebenen Bedeutungen hat, umsetzt.

6. Komplexverbindung, enthaltend eine Verbindung nach Anspruch 1 und mindestens ein Zentralatom.

7. Komplexverbindung nach Anspruch 6, dadurch gekennzeichnet, daß das Zentralatom ausgewählt wird aus:
$^{111}In$, $^{68}Ga$, $^{67}Ga$, $^{90}Y$, $^{153}Sm$, $^{52}Fe$, $^{99m}Tc$, $^{186}Re$, $^{188}Re$, $^{153}Gd$, Gd, Fe, Mn.

8. Konjugat, bestehend aus mindestens einer Komplexverbindung gemäß Anspruch 6 oder 7 und einer Transportsubstanz.

9. Konjugat nach Anspruch 8, dadurch gekennzeichnet, daß die Transportsubstanz ausgewählt wird aus: Antikörpern, Antikörperfragmenten, Proteinen, Steroiden, Lipiden, Enzymen, Zuckern, Polymeren.

10. Verfahren zur Markierung von Transportsubstanzen mit Radionukliden dadurch gekennzeichnet, daß
a) die zu markierende Transportsubstanz mit einer Verbindung der Formel I gemäß Anspruch 1 umgesetzt wird und das erhaltene Produkt dann mit dem Radionuklid versetzt wird, oder
b) die Verbindung der Formel I gemäß Anspruch 1 zunächst mit dem Radionuklid versetzt wird und anschließend das erhaltene Produkt mit der zu markierenden Transportsubstanz umgesetzt wird.

11. Verwendung der Konjugate gemäß Anspruch 8 oder 9 in der medizinischen Diagnostik.

12. Testkit, enthaltend mindestens ein Konjugat gemäß Anspruch 8 oder 9.

13. Testkit, enthaltend ein Konjugat, bestehend aus einer Verbindung der Formel I nach Anspruch 1 und einer Transportsubstanz.

Patentansprüche für folgende Vertragsstaaten: ES, GR

1. Verfahren zur Herstellung von 1,4,7,10-Tetraazacyclotridecanen der Formel I

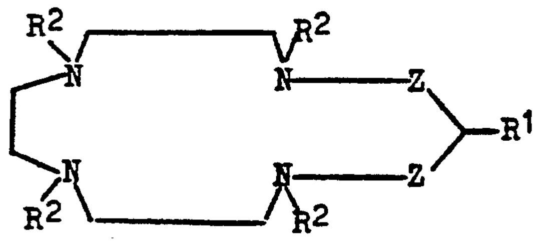

(I)

worin
Z CH$_2$ oder CO bedeutet und
$R^1$ eine Gruppe der Formel -X-NH$_2$, -X-NO$_2$, -X-NCS, -X-COOH, -X-OH, -X-N$_2^+$ oder -X-COCl oder eine Gruppe der Formel X-Hal bedeutet, wobei Hal, Fluor, Chlor, Brom oder Jod bedeutet und X eine Alkylengruppe mit 1 bis 40 C-Atomen, eine ortho-, meta- oder para-phenyl-C$_1$-C$_2$-alkylengruppe bedeutet und
$R^2$ Wasserstoff oder -(CH$_2$)$_n$-$R^3$ bedeutet, wobei
n 1 oder 2 ist und
$R^3$ -COOH, PO$_3$H$_2$, -SO$_3$H

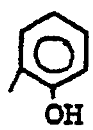 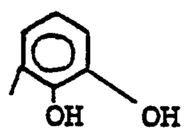 oder 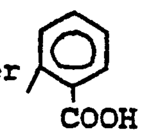

bedeutet
und wobei die vier $R^2$-Reste verschieden sind oder 2, 3 oder alle $R^2$-Reste identisch sind, dadurch gekennzeichnet, daß ein Malonsäuredialkylester der Formel II

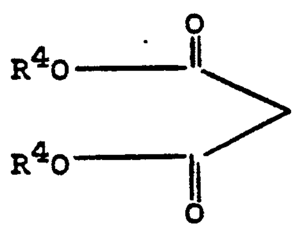

(II)

worin $R^4$ C$_1$-C$_3$-Alkyl bedeutet,
mit einer Verbindung der Formel III
A - $R^1$ (III)
worin $R^1$ die in Anspruch 1 zu Formel I angegebenen Bedeutungen hat und A Chlor oder Brom bedeutet, umsetzt
und anschließend die erhaltene Verbindung der Formel IV

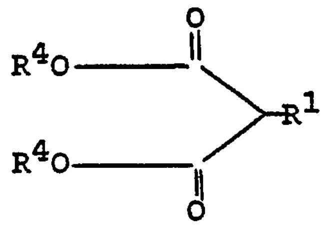

(IV)

mit Triethylentetraamin der Formel V

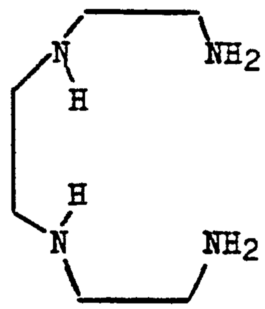

(V)

zu einer Verbindung der Formel VI

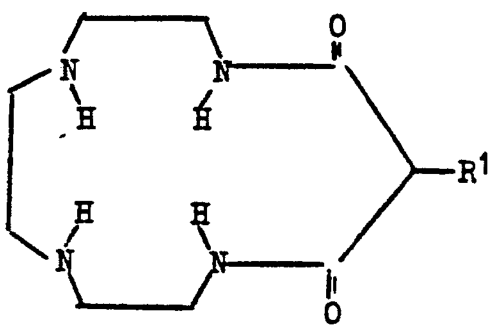

(VI)

umsetzt,

die CO- gruppen in 11,13-Position zu $CH_2$-Gruppen reduziert oder nicht reduziert und anschließend die erhaltene Verbindung der Formel VII

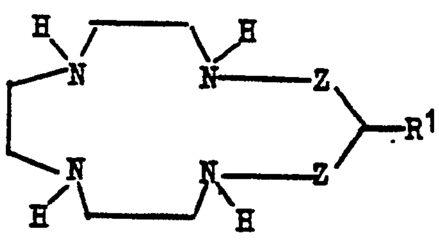

(VII)

mit Verbindungen der Formel VIII

A - $R^2$

worin A Chlor oder Brom bedeutet und $R^2$ die in Anspruch 1 zu Formel I angegebenen Bedeutungen hat, umsetzt.

2. Verfahren nach Anspruch 1, wobei mindestens eine der nachfolgenden Bedingungen erfüllt ist:

Z bedeutet $CH_2$

X bedeutet eine Alkylengruppe mit 1 bis 20 C-Atomen oder

X bedeutet eine para-Phenylengruppe oder

X bedeutet eine para-Phenylmethylengruppe
Hal bedeutet Chlor, Brom oder Jod
n ist 1
$R^3$ bedeutet -COOH, $-PO_3H_2$ oder $-SO_3H$,
3 oder alle $R^2$-Reste sind identisch.

3. Verfahren nach Anspruch 1 oder 2, wobei mindestens eine der nachfolgenden Bedingungen erfüllt ist:
Z bedeutet $CH_2$
X bedeutet eine para-Phenylengruppe oder
X bedeutet eine para-Phenylmethylengruppe oder
X bedeutet eine Alkylengruppe mit 1 bis 8 C-Atomen
Hal bedeutet Chlor, Brom oder Jod
n ist 1
$R^3$ bedeutet -COOH, oder $-PO_3H_2$,
alle $R^2$-Reste sind identisch.

4. Verfahren nach einem oder mehreren der Ansprüche 1 bis 3, dadurch gekennzeichnet, daß mindestens ein $R^2$-Substituent von Wasserstoff verschieden ist.

5. Komplexverbindung, enthaltend eine Verbindung, erhältlich nach Anspruch 1 und mindestens ein Zentralatom.

6. Komplexverbindung nach Anspruch 5, dadurch gekennzeichnet, daß das Zentralatom ausgewählt wird aus:
$^{111}In$, $^{68}Ga$, $^{67}Ga$, $^{90}Y$, $^{153}Sm$, $^{52}Fe$, $^{99m}Tc$, $^{186}Re$, $^{188}Re$, $^{153}Gd$, Gd, Fe, Mn.

7. Konjugat, bestehend aus mindestens einer Komplexverbindung gemäß Anspruch 5 oder 6 und einer Transportsubstanz.

8. Konjugat nach Anspruch 7, dadurch gekennzeichnet, daß die Transportsubstanz ausgewählt wird aus: Antikörpern, Antikörperfragmenten, Proteinen, Steroiden, Lipiden, Enzymen, Zuckern, Polymeren.

9. Verfahren zur Markierung von Transportsubstanzen mit Radionukliden dadurch gekennzeichnet, daß
a) die zu markierende Transportsubstanz mit einer Verbindung der Formel I, erhältlich gemäß Anspruch 1, umgesetzt wird und das erhaltene Produkt dann mit dem Radionuklid versetzt wird, oder
b) die Verbindung der Formel I, erhältlich gemäß Anspruch 1, zunächst mit dem Radionuklid versetzt wird und anschließend das erhaltene Produkt mit der zu markierenden Transportsubstanz umgesetzt wird.

10. Verwendung der Konjugate gemäß Anspruch 7 oder 8 in der medizinischen Diagnostik.

11. Testkit, enthaltend mindestens ein Konjugat gemäß Anspruch 7 oder 8.

12. Testkit, enthaltend ein Konjugat, bestehend aus einer Verbindung der Formel I, erhältlich nach Anspruch 1 und einer Transportsubstanz.

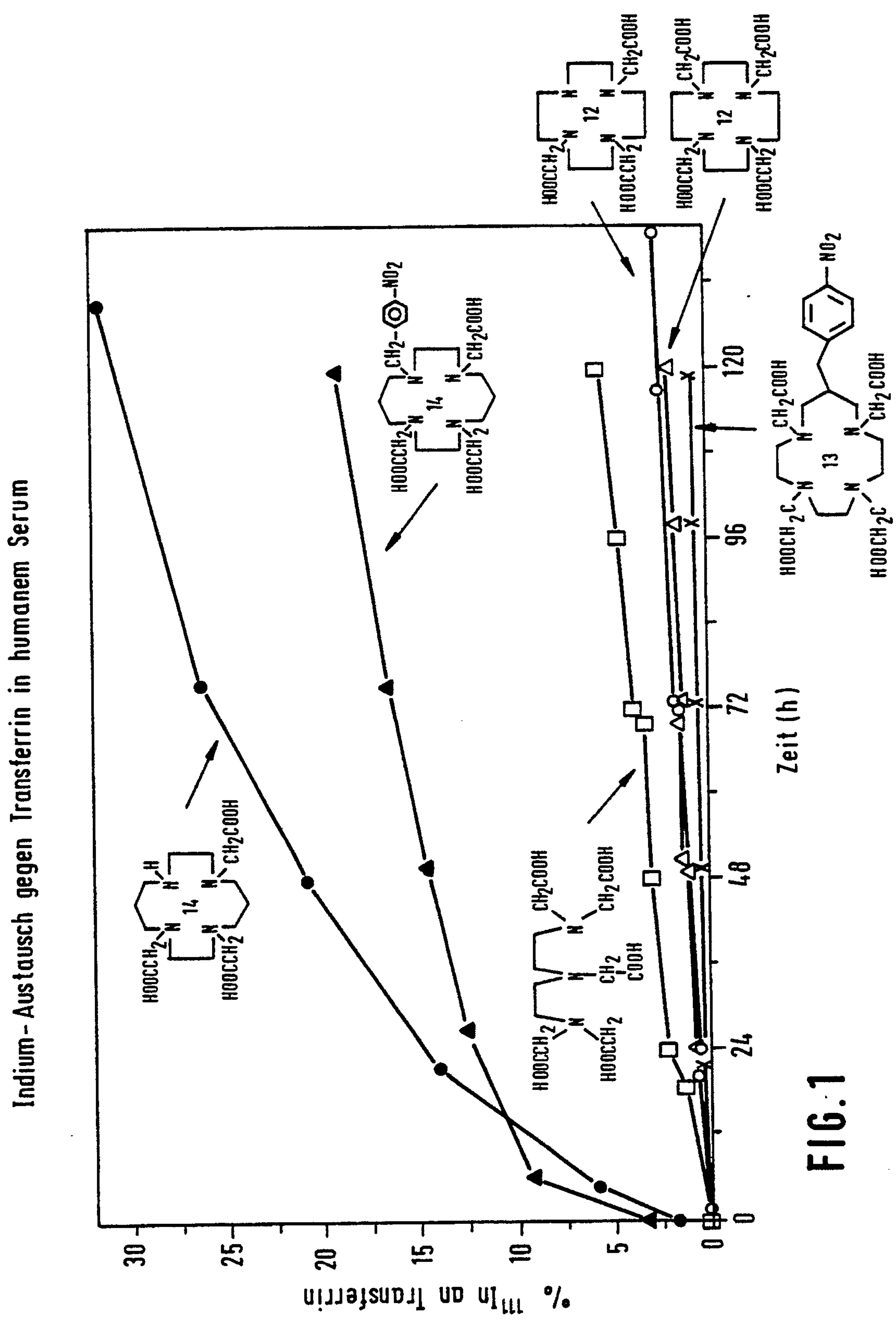

Indium-Austausch gegen Transferrin in humanem Serum
% 111In an Transferrin
0
5
10
15
20
25
30
0
24
48
72
96
120
Zeit (h)


FIG. 1

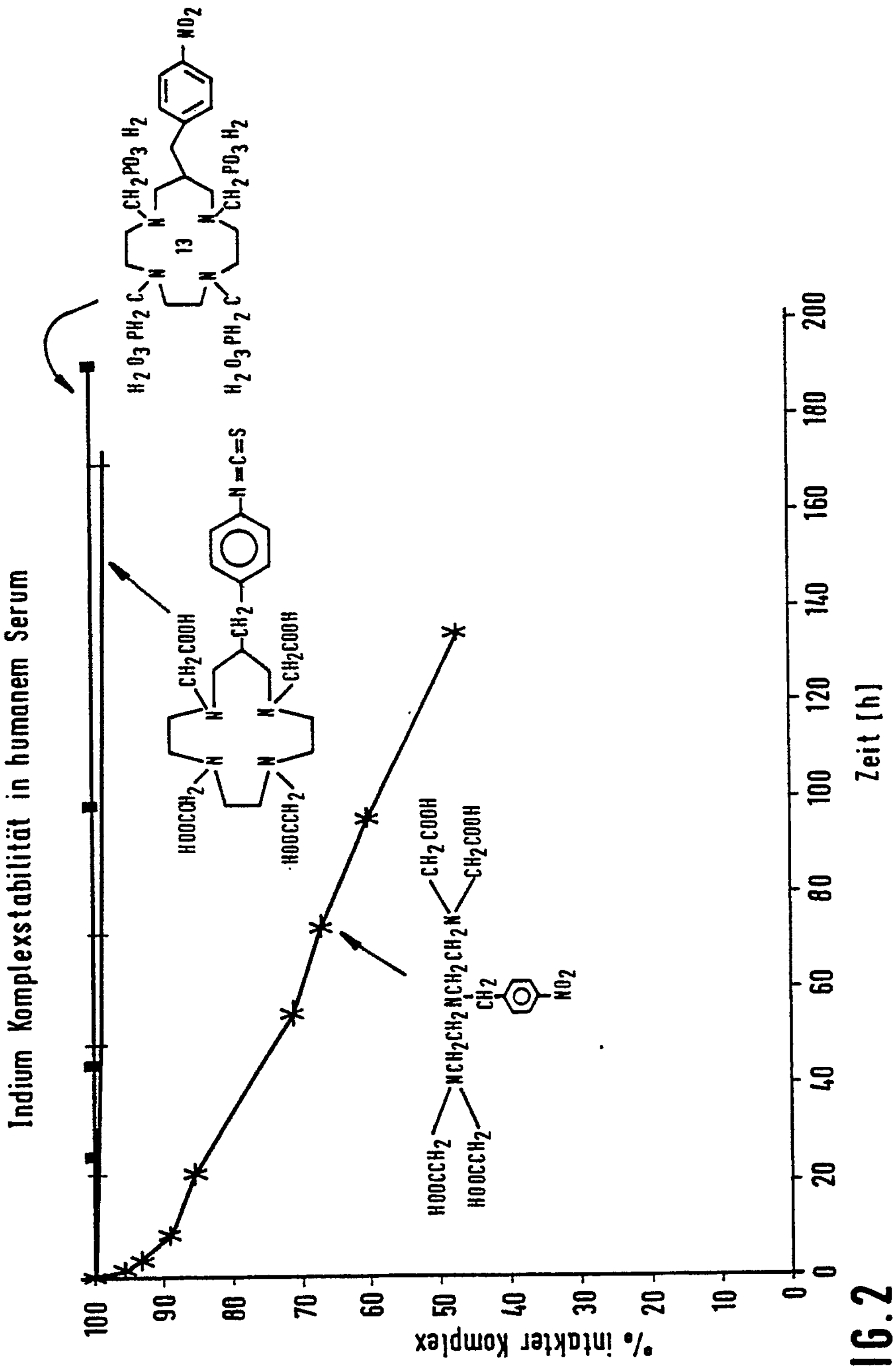
Indium Komplexstabilität in humanem Serum
% intakter Komplex
100
90
80
70
60
50
40
30
20
10
0
0
20
40
60
80
100
120
140
160
180
200
Zeit [h]

FIG. 2

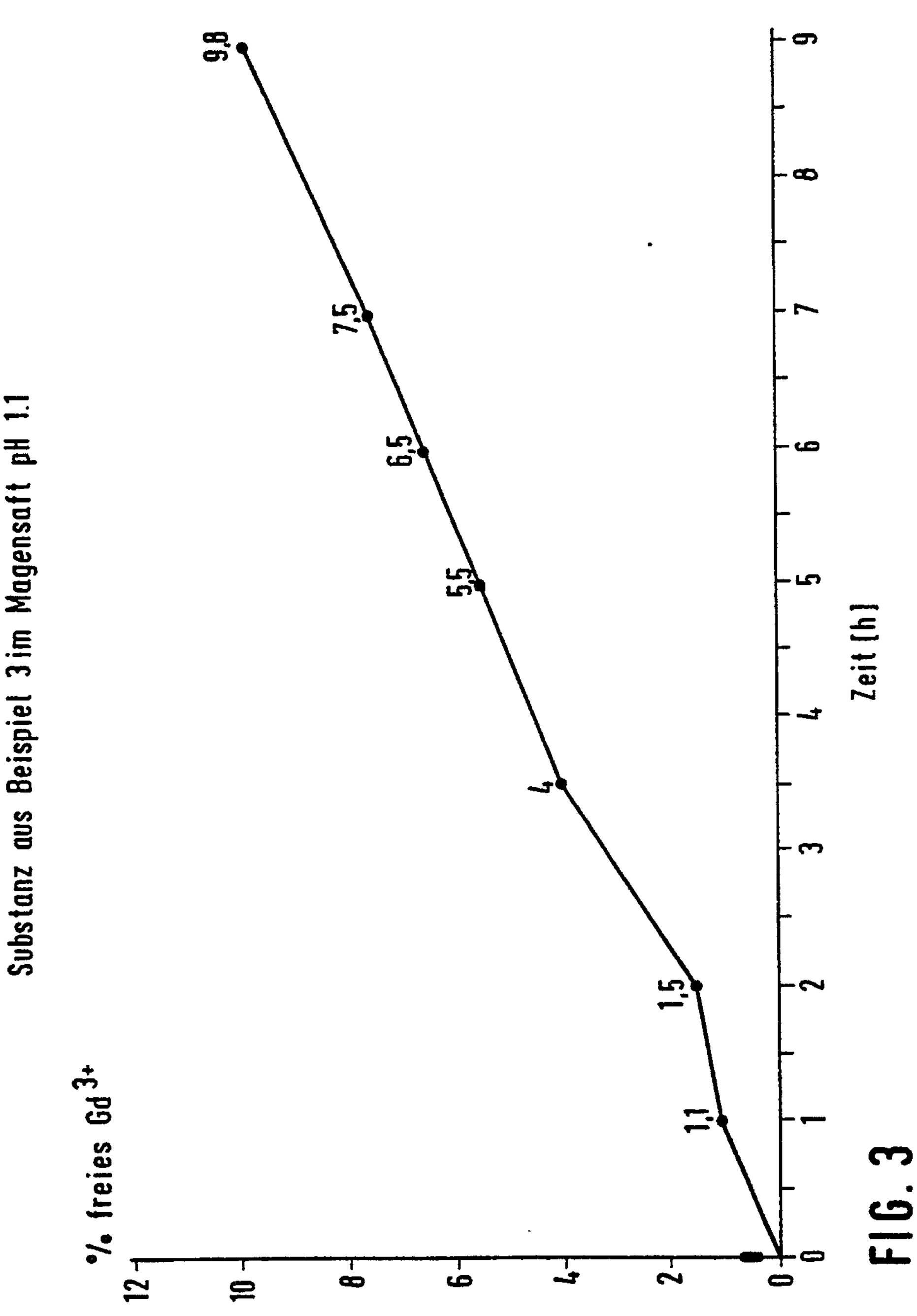
Substanz aus Beispiel 3 im Magensaft pH 1.1
% freies Gd3+
12
10
8
6
4
2
0
0
1
2
3
4
5
6
7
8
9
Zeit [h]
1,1
1,5
4
5,5
6,5
7,5
9,8

FIG. 3

## Reaktionsschema 1

FIG. 4

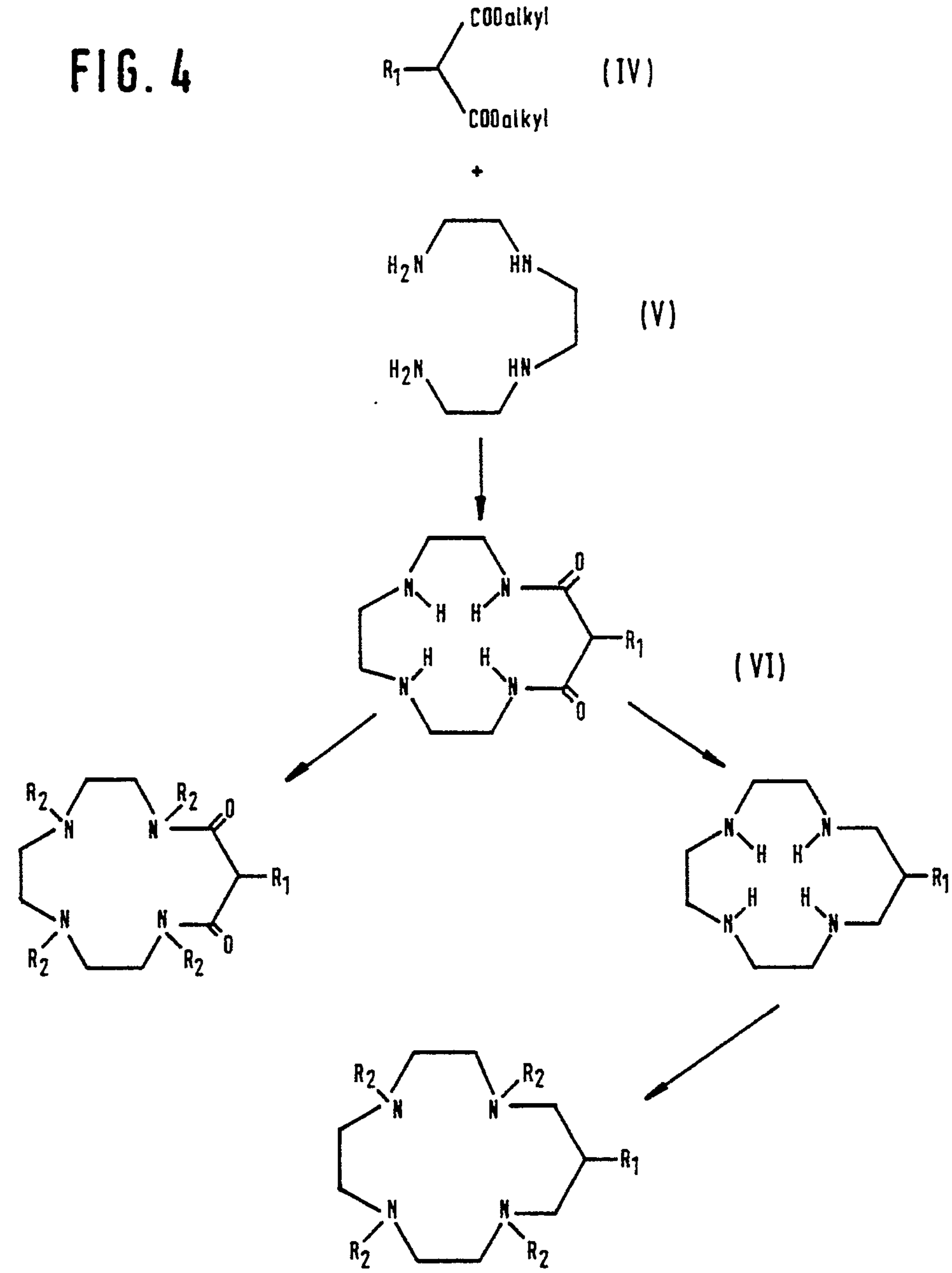

(IV)

(V)

(VI)